**Europäisches Patentamt**

**European Patent Office**

**Office européen des brevets**

(11) Veröffentlichungsnummer: **0 071 818**
**B1**

(12) **EUROPÄISCHE PATENTSCHRIFT**

(45) Veröffentlichungstag der Patentschrift:
**22.05.85**

(21) Anmeldenummer: **82106531.5**

(22) Anmeldetag: **20.07.82**

(51) Int. Cl.⁴: **A 61 F 13/08**

(54) **Thrombose-Prophylaxe-Strumpf.**

(30) Priorität: **05.08.81 DE 8122953 U**

(43) Veröffentlichungstag der Anmeldung:
**16.02.83 Patentblatt 83/7**

(45) Bekanntmachung des Hinweises auf die Patenterteilung:
**22.05.85 Patentblatt 85/21**

(84) Benannte Vertragsstaaten:
**AT CH DE FR GB LI NL SE**

(56) Entgegenhaltungen:
**DE - A - 2 458 251**
**DE - U - 1 852 501**
**DE - U - 1 929 623**
**DE - U - 7 415 893**
**DE - U - 7 420 469**

(73) Patentinhaber: **Weihermüller & Voigtmann GmbH & Co. KG, Waldsteinring 10, D-8580 Bayreuth-St. Johannis (DE)**

(72) Erfinder: **Weihermüller, Wolfgang, Waldsteinring 12, D-8580 Bayreuth (DE)**
Erfinder: **Voigtmann, Günter, Waldsteinring 10, D-8580 Bayreuth (DE)**

(74) Vertreter: **Voigt, Günter, Dipl.-Ing., Patentanwälte Dr.-Ing. Alfred Schulze, Dipl.-Ing. Günter Voigt & Partner Nordring 152, Postfach 210104, D-8500 Nürnberg 21 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf einen Thrombose-Prophylaxe-Strumpf mit im Zehenbereich vorgesehener reduzierter Kontrollöffnung und im Ristbereich erweitertem Umfang, wobei der Ristbereich bei gleicher Maschenzahl durch Vergrösserung der Maschenweite und angepasste Verlängerung des Kompressionsschussfadens im Umfang vergrössert ist.

Es sind bereits rotationssymmetrisch gestrickte Kompressionsstrümpfe mit im Ristbereich erweitertem Umfang bekannt, die sich wegen des unterbrechungslosen Strickvorganges durch ihre Preisgünstigkeit besonders auszeichnen. Nachteilig ist jedoch, dass sich die untere fussseitige Öffnung beim Tragen am Bein des Patienten oberhalb der Zehen befindet, so dass diese frei liegen und der Kompressionsstrumpf keine genügende Fixierung findet und nach oben wegrutschen kann (DE-U-7 415 893).

Bei einem anderen bekannten Kompressionsstrumpf ist während der Herstellung auf der Rundstrickmaschine eine echte Ferse eingependelt, wodurch der Strumpf eine asymmetrische Form erhält und durch die bauchig ausgebildete Ferse eine Fixierung am Fuss des Patienten möglich ist. Da es jedoch fertigungstechnisch sehr schwierig ist, beim Einpendeln der Ferse auch einen Kompressions-Gummifaden in die Maschen einzulegen, ergibt sich beim Tragen des Strumpes am Fuss des Patienten eine ungleichmässige und medizinisch ungünstige Kompressionswirkung. Auch hier liegt die untere Öffnung des schlauchförmigen Gestrickes oberhalb der Zehen des Patienten (DE-U- 7 420 469).

Schliesslich ist bei einem weiteren bekannten Thrombose-Prophylaxe-Strumpf die untere Öffnung im Zehenbereich als reduzierte Kontrollöffnung ausgebildet, so dass die Zehen des Patienten verdeckt sind und der Strumpf gleichzeitig fixiert ist. Im Ristbereich ist dieser Strumpf zunächst symmetrisch und mit vergrösserter Maschenweite gefertigt, wobei durch eine nachträgliche mechanisch-thermische Behandlung durch eine Dehnung der Maschen eine sogenannte falsche Ferse ausgearbeitet ist. Dadurch wird zwar im Ristbereich das Einpedeln einer echten Ferse erspart, jedoch erhöhen sich durch die nachträgliche Bearbeitung die Herstellungskosten (Versuche im Hause der Anmelderin).

Aufgabe der Erfindung ist es deshalb, einen Thrombose-Prophylaxe-Strumpf mit im Zehenbereich vorgesehener reduzierter Kontrollöffnung zur Verfügung zu stellen, bei dem die vorgenannten Nachteile weitgehend vermieden sind und trotz kurzer Maschinenlaufzeit der Kompressionsstrumpf beim späteren Tragen ähnliche Eigenschaften aufweist wie ein Strumpf mit echter eingepedelter Ferse, so dass der Strumpf hinreichend fixiert werden kann.

Diese Aufgabe wird bei einem Thrombose-Prophylaxe-Strumpf der eingangs genannten Gattung mit der im Patentanspruch gekennzeichneten Merkmalskombination und der beschriebenen Mittel gelöst.

Einzelheiten der Erfindung und ihre Vorteile sind im folgenden anhand eines Ausführungsbeispiels näher beschrieben und in der Zeichnung dargestellt.

Fig. 1 zeigt den unteren Teil des neuen Strumpfes mit Blick auf die Fussunterseite;

Fig. 2 zeigt den Strumpf nach Fig. 1 in der Seitenansicht.

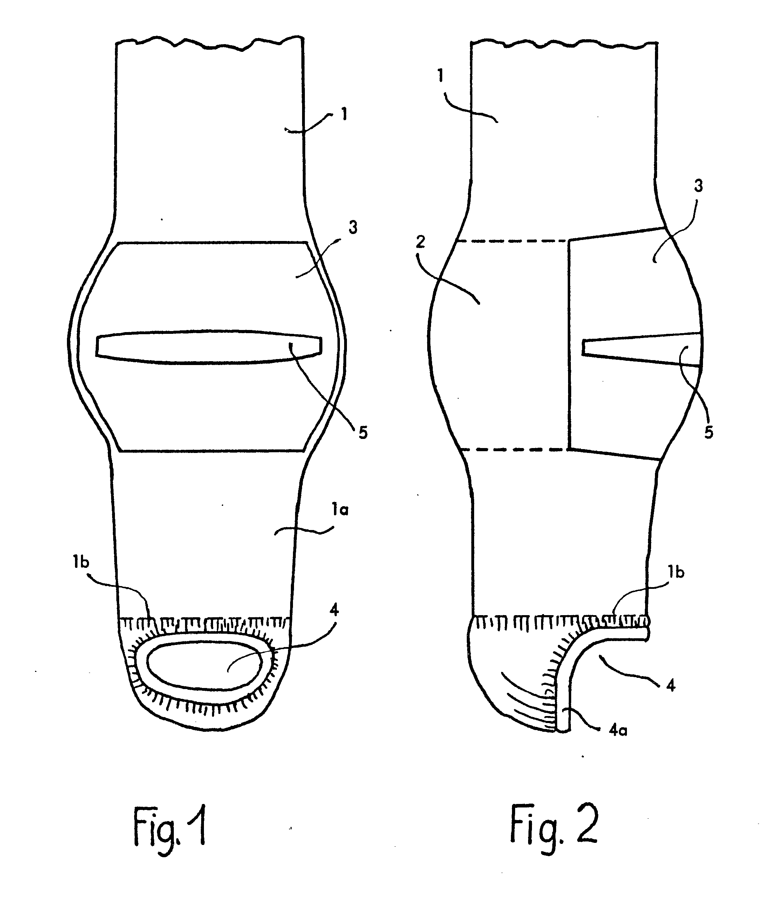

Wie aus den Figuren 1 und 2 erkennbar ist, ist der Thrombose-Prophylaxe-Strumpf 1 bei der Herstellung auf der Rundstrickmaschine im Ristbereich 2 durch Vergrösserung der Maschenweite und durch angepasste Verlängerung des in die Maschen eingelegten Kompressions-Schussfadens im Umfang vergrössert, wobei die übrigen Bereiche des Strumpfes 1 und 1a in bekannter Weise ausgeführt und mit Kompressions-Schussfäden versehen sind, die den medizinischen Anforderungen entsprechen. Unterhalb der Maschenreihe 1b ist der Kompressions-schussfaden weggelassen und das üblicherweise schlauchförmige offene Ende ist durch Veränderung des Strichschemas als reduzierte Kontrollöffnung 4 ausgebildet, deren Rand noch mit einem Verstärkungsband 4a eingefasst sein kann. Beim Tragen des Strumpfes sind die Zehen des Patienten verdeckt und die Kontrollöffnung 4 liegt etwa an der Unterseite der Zehen. Der mit erweitertem Umfang hergestellte Ristbereich 2 ist an dem der Ferse zugeordneten Flächenabschnitt 3 etwa bis zur Hälfte des Umfanges des Strumpfes zur Vertärkung mit einem zusätzlichen Faden gestrickt, der dem in den übrigen Bereichen 1 und 1a des Strumpfes verwendeten Faden gleich oder ähnlich ist. Etwa in der Mitte des Flächenabschnittes 3 der verstärkten Ferse sind mit Hilfe eines unterscheidbaren anderen Fadens etwa 2 bis 20 Maschenreihen eingependelt, die sich über ein Viertel bis einen halben Umfang des Gestrickes erstrecken können. Im Vergleich zum Einpendeln der üblichen echten Ferse werden für das Einstricken des erfindungsgemässen Streifens 5 nur vergleichsweise wenige Pendelungen ausgeführt, so dass der übliche Rundstrickvorgang nur kurzzeitig unterbrochen wird. Da für die Herstellung des schmalen Streifens 5 im verstärkten Flächenabschnitt 3 der Ferse ein andersartiger, ggf. auch andersfarbiger, Faden verwendet wird, ist der Fersenbereich des Strumpfes für das richtige Anlegen eindeutig festgelegt, während sich durch den eingearbeiteten schmalen Streifen 5 im praktischen Gebrauch die Wirkung einer Quasi-Ferse ergibt.

Ein solcher Thrombose-Prophylaxe-Strumpf ist herstellungstechnisch schneller und kostengünstiger zu produzieren, zeigt auch im Ristbereich eine optimale Druckverteilung und weist auch sonst die geschätzen Vorzüge der üblichen Kompressionsstrümpfe mit reduzierter Kontrollöffnung auf.

## Patentanspruch

Thrombose-Prophylaxe-Strumpf (1) mit im Zehenbereich vorgesehener reduzierter Kontrollöffnung (4) und im Ristbereich (2) erweitertem Umfang, wobei der Ristbereich (2) bei gleicher Maschenzahl durch Vergrösserung der Maschenweite und angepasste Verlängerung des Kompressionsschussfadens im Umfang vergrössert ist, dadurch gekennzeichnet, dass der im Umfang vergrösserte Ristbereich (2) in dem der Ferse zugeordneten Flächenabschnitt (3) mit einem zusätzlichen, dem übrigen Gestrickfaden ähnlichen Faden verstärkt ist und, vorzugsweise in der Mitte des verstärkten Flächenabschnitts (3) der Ferse, mit Hilfe eines unterscheidbaren anderen Fadens zur Fixierung der Ferse in Richtung des Strumpfumfangs zusätzlich ein schmaler Streifen (5) in Höhe von etwa 2 bis 20 Maschenreihen und in der Breite von einem Viertel bis einer Hälfte des Ristbereich-Umfangs eingestrickt ist.

## Claim

1. Thrombosis preventive stocking (1) with provision for a reduced control opening (4) in the toe area and enlarged circumference in the instep area (2), the latter involving the same number of stitches, with greater mesh size and matched extension of the pressure weft yarn to enlarge the instep circumference, wherein the instep area (2), enlarged in circumference is reinforced in that section of it (3) assigned to the heel, by an additional thread similar to the other knitted threads, and wherein, preferably in the centre of the reinforced area of the heel (3), with the aid of another distinguishable thread to secure the heel in the direction of the stocking circumference, a narrow strip (5) is knitted additionally at a height of approximately (2 to 20) mesh rows and at a width of a quarter to a half of the instep circumference.

## Revendication

Bas prophylactique (1) contre la thrombose avec une ouverture de contrôle réduite (4) dans la zone des doigts de pied et une périphérie élargie dans la zone du dos de pied, ledit élargissement étant obtenu par une augmentation de la largeur des mailles et une prolongation adaptée du fil de trame à effet comprimant alors que le nombre de mailles reste constant, caractérisé en ce que le segment de surface (3) destiné au talon, prévu dans la partie du bas à périphérie élargie, situé dans la zone du dos de pied, est renforcé par un fil supplémentaire, similaire au fil à tricoter utilisé pour le bas et en ce que de préférence au milieu du segment de surface renforcé destiné au talon, est insérée une étroite bande (5) supplémentaire d'un fil différent, distinguable, d'une hauteur d'environ (2 à 20) rangs et d'une largeur d'environ un quart à la moitié de la périphérie de la zone du dos de pied, afin de permettre la détermination de l'emplacement par rapport à la périphérie du bas.

Fig.1          Fig.2